Europäisches Patentamt

European Patent Office

Office européen des brevets

(11) Veröffentlichungsnummer: **0 222 979 B1**

# EUROPÄISCHE PATENTSCHRIFT

(45) Veröffentlichungstag der Patentschrift: **08.07.92**

(51) Int. Cl.⁵: **A61F 2/36**

(21) Anmeldenummer: **86110508.8**

(22) Anmeldetag: **30.07.86**

(54) **Schaftteil eines Hüftgelenkes.**

(30) Priorität: **06.08.85 DE 3528151**

(43) Veröffentlichungstag der Anmeldung:
**27.05.87 Patentblatt 87/22**

(45) Bekanntmachung des Hinweises auf die
Patenterteilung:
**08.07.92 Patentblatt 92/28**

(84) Benannte Vertragsstaaten:
**AT BE CH DE FR GB IT LI LU NL SE**

(56) Entgegenhaltungen:
**EP-A- 0 098 224      EP-A- 0 145 939**
**DE-B- 2 839 093      DE-U- 8 411 765**
**FR-A- 1 278 359      FR-A- 2 344 272**
**FR-A- 2 433 932**

(73) Patentinhaber: **GMT Gesellschaft für medizini-**
**sche Technik mbH**
**Holstenstrasse 2**
**W-2000 Hamburg 50(DE)**

(72) Erfinder: **Engelbrecht, Eckart, Dr. med.**
**Andreasstrasse 33**
**W-2000 Hamburg 60(DE)**
Erfinder: **Hahn, Michael**
**Niendorfer Kirchenweg 7F**
**W-2000 Hamburg 61(DE)**

(74) Vertreter: **Heldt, Gert, Dr. Dipl.-Ing.**
**Neuer Wall 59 III**
**W-2000 Hamburg 36(DE)**

## Beschreibung

Die Erfindung betrifft einen Schaftteil eines Hüftgelenkes mit einem im wesentlichen kugeligen Schaftkopf und einem sich über einen Kragen an diesen anschließenden Schaft, der im Anschluß an den Kragen in Richtung auf sein unteres Ende eine Krümmung aufweist und mittels einer als Zugstange ausgebildeten Spannvorrichtung in einer Markhöhle eines Oberschenkelknochens befestigbar ist.

Aus der FR-A-243 3 932 ist ein solcher Schaftteil bekannt, der zementlos in einer Markhülle eines Oberschenkelknochens befestigt wird. Die Befestigung erfolgt mit Hilfe von Schraubverbindungen, die sich vom Kragen der Prothese in Richtung auf Verstärkungsplatten erstrecken, die lateral und medial den Femurknochen stärken. Diese Schraubverbindungen spannen den Kragen der Prothese in diesen Platten, die außen auf dem Femurknochen besfestigt sind. Zur Befestigung der Platten auf dem Femurknochen sind Schraubverbindungen vorgesehen, die als Knochenschrauben ausgebildet sind und durch entsprechende Löcher der Platten hindurchragen und diese mit dem Knochen verbinden.

Der Kragen des Schaftteiles liegt mit seiner Unterkante formschlüssig auf der entsprechenden Resektionsfläche des Knochens auf. Sollte die Markhöhle des Femurknochens nicht sorgfältig genug dem Schaft angepaßt sein, so daß dieser innerhalb der Markhöhle ein, wenn auch nur geringes Spiel aufweist, womit zu rechnen ist, dann kann eine Bewegung der Prothese innerhalb der Markhöhle nur durch einen festen Sitz des Kragens auf der Resektionsfläche unterbunden werden. Da jedoch diese Auflagefläche vergleichsweise klein ist, und außerdem eine Verpressung der Kragenunterseite mit dieser Sektionsfläche nur an zwei einander gegenüberliegenden Stellen des Kragens erfolgt, muß damit gerechnet werden, daß sich der ohnehin recht kurze Stiel innerhalb der Markhöhle unter den erheblichen Wechselbelastungen, die auf den Hüftkopf einwirken, sehr bald lockert. Dadurch entstehen Schmerzen für den Patienten und sehr bald ist mit einer vollkommenen Lockerung und Funktionsunfähigkeit des gesamten Schaftteiles zu rechnen. Dieser besitzt auf seiner gesamten Länge keine Ausbildung, die eine formschlüssige Verbindung des Stiels mit dem ihm umgebenden Knochen herbeiführen könnte.

Aufgabe der vorliegenden Erfindung ist es daher, den Schaftteil eines Hüftgelenkes der einleitend genannten Art so zu verbessern, daß eine Übertragung der in den Schaft eingeleiteten Kräfte über den gesamtem Querschnitt des Schaftes weitgehend gleichmäßig gewährleistet ist.

Diese Aufgabe wird erfindungsgemäß dadurch gelöst, daß der Schaft in seiner Oberfläche sich in seiner Längsrichtung geradlinig erstreckende Rillen zur formschlüssigen Verbindung mit der die Schaftoberfläche umgebenden Knochenmasse aufweist, und daß der Schaft an seinem dem Kragen benachbarten oberen Ende in der Markhöhle mit der als Zugstange ausgebildeten Spannvorrichtung verspannbar ist, welche Spannvorrichtung im enstpannten Zustand gegenüber dem Schaft einerseits und der Markhöhle andererseits abstützbar ist.

Die formschlüssige Verbindung der Schaftoberfläche mit der umgebenden Knochenmasse sorgt dafür, daß eine Lockerung des Schaftteils innerhalb der Markhöhle vermieden wird. Die Verspannung des Schaftteiles der Markhöhle führt zu einer festen Verankerung und damit zu einer weitgehend gleichmäßigen Druckbelastung des Schaftes innerhalb der Markhöhle. Diese gleichmäßige Druckbelastung fördert im übrigen die Ausbildung von Knochengewebe, das sich gleichmäßig um den gesamten Schaft ausbildet. Auf diese Weise kann ein Ausgleich zwischen den Zonen höherer Druckbelastung und niedriger Druckbelastung erfolgen.

Gemäß einer bevorzugten Ausführungsform der Erfindung verlaufen in der Oberfläche in Längsrichtung des Schaftes sich erstreckende Rillen. Diese Rillen vergrössern die Oberfläche des Schaftteils erheblich und verbessern damit eine innige Verbindung zwischen der Markhöhle und der Oberfläche des Schaftes.

Diese innige Verbindung verhindert eine Lockerung des Schaftes. Außerdem wird durch die Rillung die Oberfläche des Schaftes vergrößert, so daß dadurch eine Verminderung der Flächenpressung eintritt.

Gemäß einer weiteren bevorzugten Ausführungsform der Erfindung ist der Schaft an seinem dem Kragen benachbarten oberen Ende mit einer Spannvorrichtung in einer ihn umgebenden Markhöhle eines Oberschenkelknochens verspannt. Diese Verspannung liegt in einem Bereich, in dem bei einem unverspannten Schaft nur geringe Kräfte vom Schaft auf den Oberschenkel übertragen werden. Die Verspannung gleicht damit diese ungleiche Druckbelastung des Schaftes aus, so daß von der Verspannung ein den Schaft in der Markhöhle aufrichtendes Moment erzeugt wird. Dieses Moment sorgt dafür, daß die im inneren Bereich der Krümmung auftretenden Druckkräfte vermindert und von dem äußeren Bereich des Schaftes auf den Knochen übertragen werden. Dieser Druckausgleich entsteht durch einen in Richtung auf den Prothesenkopf verlaufenden Kraftschluß. Die gleichmäßige Belastung der Oberfläche des Schaftes über dessen gesamten Querschnitt führt dazu, daß Lockerungen des Schaftes innerhalb der Markhöhle vermieden werden.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

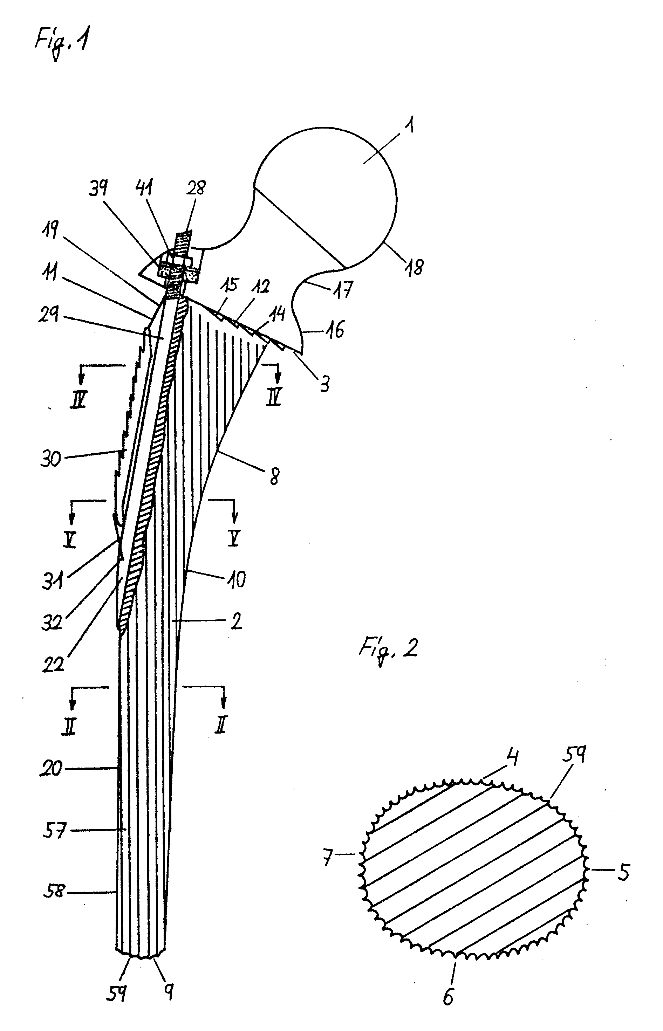

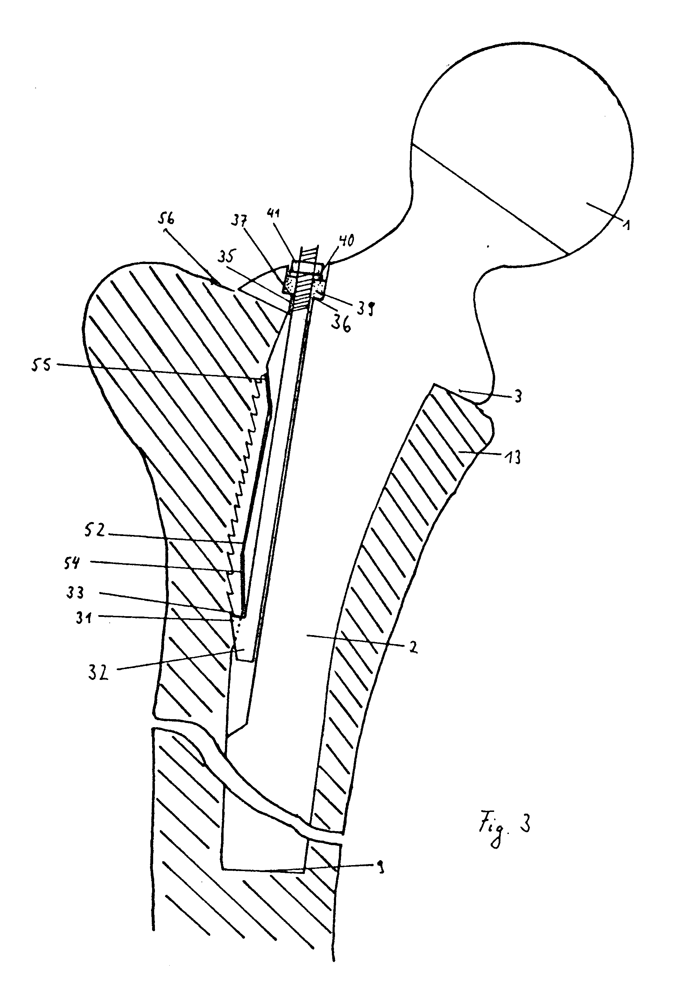

Fig. 1: eine Seitenansicht eines Schaftteils,

Fig. 2: einen Querschnitt durch einen Schaftteil entsprechend der Schnittlinie II-II in Figur 1 in vergrößerter Darstellung,

Fig. 3: einen Längsschnitt durch einen Oberschenkelknochen mit eingebautem Schaftteil,

Fig. 4: einen Querschnitt durch einen Schaftteil entsprechend der Schnittlinie IV-IV in Figur 1,

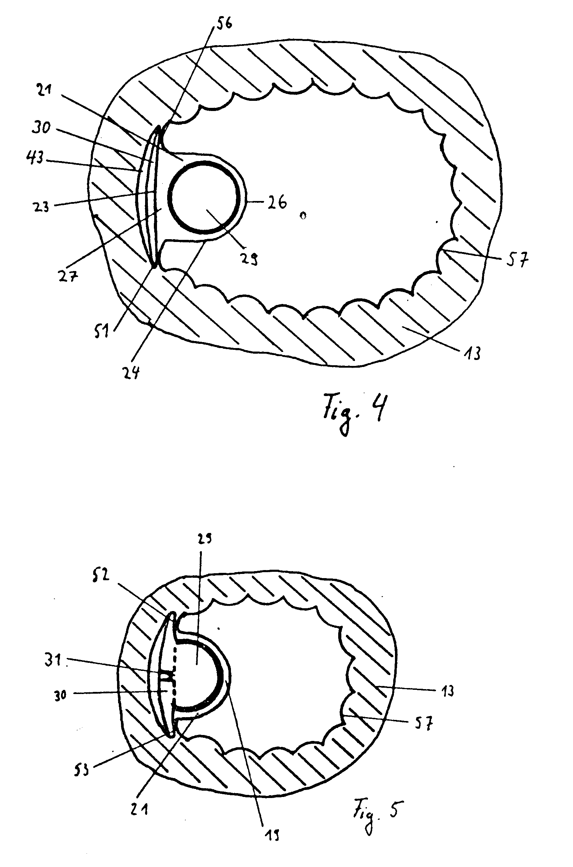

Fig. 5: einen Querschnitt durch einen Schaftteil entsprechend der Schnittlinie V-V in Figur 1,

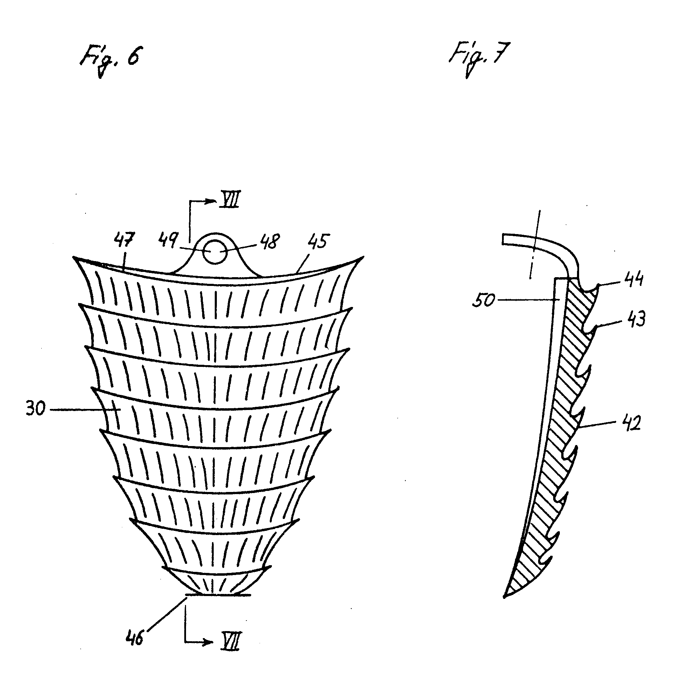

Fig. 6: eine räumliche Darstellung eines Schildes und

Fig. 7: einen Längsschnitt durch ein Schild entsprechend der Schnittlinie VII-VII in Figur 6

Ein Schaftteil eines Hüftgelenkes besteht im wesentlichen aus einem im wesentlichen kugeligen Schaftkopf 1 und einem Schaft 2. Der Schaft 2 ist mit dem Schaftkopf 1 fest verbunden und schließt sich an diesen unmittelbar im Anschluß an einen Kragen 3 an, der den Schaftkopf 1 gegenüber dem Schaft 2 begrenzt. Der Schaft 2 ragt etwa zentrisch aus dem Schaftkopf 1 heraus, so daß der Kragen 3 auf allen Seiten 4,5,6,7 des Schaftes 2 etwa gleichweit über die den Schaft 2 begrenzenden Seiten 4,5,6,7 heraussteht. Dabei bildet der Kragen 3 auf seiner dem Schaft 2 zugewandten Unterseite eine Ebene aus, die in etwa senkrecht aufeinander gegenüberliegenden Seiten 4,6 des Schaftes 2 steht. Diese Seiten 4,6 stellen einander gegenüberliegende breitere Begrenzungen des Schaftes 2 dar, während die von ihnen eingeschlossenen Seiten 5,7 einandergegenüberliegende schmalere Begrenzungen des Schaftes 2 bilden. Sie begrenzen den Schaft 2 im Bereich von Krümmungsradien einer Krümmung 8, die der Schaft 2 in Richtung auf sein dem Schaftkopf 1 gegenüberliegendes Ende 9 durchläuft. Dabei neigt sich der Schaftkopf 1 in Richtung auf einen inneren Krümmungsradius 1o, der kleiner ist als ein ihm gegenüberliegender äußerer Krümmungsradius 11. Der innere Krümmungsradius 1o mündet unter einem Winkel in den Kragen 3 ein, der größer ist als ein rechter Winkel, während der äußere Krümmungsradius 11 einen Winkel mit dem Kragen 3 ausbildet, der kleiner ist als 9o°. Auf dem Kragen 3 erheben sich in Richtung des Schaftes 2 Zähne 12, die der Befestigung des Schaftteils in einem Oberschenkelknochen 13 dienen. Diese Zähne 12 verlaufen in etwa parallel zueinander und besitzen auf ihrer dem inneren Krümmungsradius 1o zugewandten unteren Fläche 14 einen steileren Anstieg als auf ihrer der unteren Fläche 14 abgewandten oberen Fläche 15. Die Zähne 12 stehen in etwa quer zu einer gedachten Ebene, die durch den inneren Krümmungsradius 1o und den äußeren Krümmungsradius 11 verläuft.

Auf seiner dem Schaftkopf 1 zugewandten Oberseite 16 mündet der Kragen 3 in eine ringförmigen Mulde 17 ein, aus der sich der Schaftkopf 1 mit seiner kugeligen Oberfläche 18 erhebt. Diese kugelige Oberfläche 18 ist in einer nicht dargestellten Hüftpfanne drehbar gelagert, die in einem ebenfalls nicht dargestellen Beckenknochen eingelassen ist.

Im Bereich des äußeren Krümmungsradius 11 erstreckt sich durch den Schaft 2 eine Ausnehmung 19, die in Form einer Sekante sich durch den äußeren Krümmungsradius 11 erstreckt. Diese Sekante beginnt in etwa im Bereich der Einmündung des äußeren Krümmungsradius 11 in den Kragen 3 und endet in einem Bereich, in dem der äußere Krümmungsradius 11 in ein sich in Richtung auf das untere Ende 9 des Schaftteils erstreckendes grades Endstück 2o einmündet. Dieses gerade Endstück 2o besitzt eine Länge, die in etwa 1,5 mal größer als die Außenkrümmung 11 ist. Die Ausnehmung 19 ist als eine Höhlung 21 ausgebildet, die auf ihrer dem äußeren Kürmmungsradius 11 zugewandten Seite offen ist. Diese Höhlung 21 besitzt in ihrem Querschnitt eine unterschiedliche Tiefe. Diese Tiefe ist im Bereich des Kragens 3 und an einem dem Kragen gegenüberliegenden Ende 22 der Höhlung 21 am kleinsten. Zwischen dem Kragen 3 und dem Ende 22 durchschreitet die Tiefe der Höhlung 21 ein Maximum 23. Im Bereich dieses Maximums 23 sind einander gegenüberliegende Seitenwandungen 24, 25 der Höhlung 21 am höchsten ausgebildet. Diese Seitenwandungen 24, 25 laufen im Inneren des Schafts 2 zu einem Kreisbogen 26 zusammen, der einer Öffnung 27 der Ausnehmung 19 gegenüberliegt. In dieser Höhlung 21 wird eine Spannvorrichtung 28 geführt. Diese besteht im wesentlichen aus einer Zugstange 29 und einem Schild 3o, das über eine Mitnehmerraste 31 an die Zugstange 29 angekoppelt ist. Diese Mitnehmerraste 31 ist an der Zugstange 29 befestigt. Die Befestigung der Mitnehmerraste 31 erfolgt an einem dem Kragen 3 abgewandten unteren Ende 32 der Zugstange 29. Die Mitnehmerraste 31 ist in Form eines Zahnes 33 ausgebildet, der sich in einer vom Kreisbogen 26 abgewandten Richtung auf der Mitnehmerraste 31 erstreckt. Dieser Zahn 33 erhebt sich in eine vom Ende 32 abgewandte Richtung der Zugstange 29 aufwärts.

Die Zugstange kann beispielsweise zylindrisch ausgebildet sein und ragt mit ihrem dem unteren Ende 32 abgewandten oberen Ende 34 durch eine im Kragen 3 vorgesehene Bohrung 35. Diese Bohrung 35 erstreckt sich in Richtung der Höhlung 21 durch den Kragen 3.

Auf ihrem der Höhlung 21 abgewandten oberen Seite 36 ist die Bohrung 35 zu einer Sitzfläche 37 erweitert. Diese Sitzfläche ist in den Kragen 3 soweit eingelassen, daß innerhalb des Kragens 3 seitliche Wandungen 38 ausgebildet sind, an denen eine elastische Zwischenlage 39 geführt ist. Diese elastische Zwischenlage 39 kann beispielsweise aus einem Silikon bestehen. Durch diese elastische Zwischenlage 39 ragt das obere Ende 34 der Zugstange 29 hindurch und stützt sich über eine Scheibe 4o mit einer Spannmutter 41 auf der elastischen Zwischenlage 39 ab. Die Bohrung 35 umgibt die Zugstange 29 mit einem Spiel, das groß genug ist, Bewegungen der Zugstange 29 zuzulassen, die quer zur Längsrichtung der Zugstange verlaufen. Derartige Bewegungen werden von der elastischen Zwischenlage 39 aufgenommen. Zweckmäßigerweise ist die Bohrung 35 in Form eines sich in Richtung auf die Höhlung 21 erweiternden Kegels ausgebildet, so daß die Zugstange um ihr oberes Ende 34 Schwenkbewegungen innerhalb derHöhlung 21 ausführen kann.

Der Schild 3o ist auf seiner der Zugstange 29 abgewandten Außenfläche 42 mit Zähnen 43 versehen, die sich in einander parallel verlaufenden Reihen quer zur Längsrichtung der Zugstange 29 erstrecken. Diese Zähne 43 erheben sich mit ihren Schneidkanten 44 in Richtung auf das obere Ende 34 der Zugstange 29. Dabei ist der Schild 3o in Form eines Kreisbogens um die Zugstange 29 gewölbt. Es erhält auf diese Weise eine Wölbung 45, die eine großflächige Anlage des Schildes 3o an einer für die Aufnahme des Schaftes 2 vorbereiteten Markhöhle gestattet.

Mit seinem dem oberen Ende 34 abgewandten unteren Ende 46 liegt der Schild 3o auf der Mitnehmerraste 31 auf. In Richtung auf sein oberes Ende 47 erweitert sich der Schild 3o schildförmig. Am oberen Ende 47 ist eine Öse 48 vorgesehen, die in etwa auf einer sich durch den Schild 3o erstreckenden Mittellinie liegt. Durch diese Öse 48 erstreckt sich eine Bohrung 49, deren Querschnitt so bemessen ist, daß die Zugstange 29 durch sie hindurch mit Spiel verlaufen kann.

Beidseits der Höhlung 21 liegt der Schild 3o mit Auflageflächen 5o, 51 auf dem Schaft 2 auf. Entlang dieser Auflageflächen 51 gleitet der Schild 3o in Längsrichtung des Schaftes 2, wenn er mit Hilfe der Zugstange 29 in Richtung auf den Kragen 3 angehoben wird. Zu diesem Zwecke sind auf dem Schaft 2 Druckflächen 52, 53 ausgebildet, auf denen die Auflageflächen 5o, 51 aufliegen. Diese Druckflächen 52, 53 sind an mindestens zwei Stellen als schiefe Ebenen 54, 55 ausgebildet, die sich in Richtung auf den Schild 3o erheben. Die Erhebungen der schiefen Ebenen 54, 55 verlaufen in Richtung auf den Kragen 3. Wird der über die Mitnehmerraste 31 an die Zugstange 29 angekoppelte Schild 3o mit dieser in Richtung auf den Kragen 3 angehoben, so läuft der Schild 3o mit seinen Auflageflächen 5o, 51 auf die auf den Druckflächen 52, 53 aufgebrachten schiefen Ebenen 54, 55 auf, so daß der Schild 3o eine von der Zugstange 29 weggerichtete Kippbewegung um ihr unteres Ende 46 vollzieht. Sie wird dabei in Längsrichtung der Zugstange 29 an der Öse 48 geführt. Durch diese Kippbewegung erhalten die Zähne 43 eine Neigung in Richtung auf die sich durch den Oberschenkelknochen 13 erstreckende Markhöhle, in deren Wandungen sie sich beim weiteren Anziehen der Spannmutter 41 eingraben können. Auf diese Weise wird über den Schild 3o und die Zugstange 29 eine den Schaft 2 in der Markhöhle verspannende Spannkraft von der Markhöhle auf den Schaft 2 ausgeübt.

Darüber hinaus liegt der Kragen 3 mit den Zähnen 12 fest auf einer am Oberschenkelknochen 13 vorbereiteten Auflagefläche 56 an. Diese Zähne 12 graben sich in die Auflagefläche 56 ein und verhindern auf diese Weise, daß der Schaftteil innerhalb der Markhöhle des Oberschenkelknochens 13 Schwenkbewegungen um sein Ende 9 ausführen kann. Schließlich sind zur weiteren Führung des zementlos in der Markhöhle verankerten Schaftes 2 Rillen 57 im Schaft 2 vorgesehen. Diese Rillen 57 verlaufen in Längsrichtung des Schaftes 2 einander parallel. Dabei ist als Längsrichtung des Schaftes 2 die Richtung des unteren Endstückes 2o vorgesehen, zu deren dem äuseren Krümmungsradius 11 zugewandten Außenfläche 58 die Rillen 57 parallel verlaufen. Dabei besitzen die Rillen 57 einander etwa gleichbleibende Breiten. Zwischen den einzelnen Rillen 57 sind scharfe Kanten 59 ausgebildet, die sich in den Oberschenkelknochen 13 hineinschneiden, wenn der Schaft 2 in die Markhöhle eingesetzt wird. Auf diese Weise erhält der Schaft 2 eine feste Ausrichtung bezüglich des Oberschenkelknochens 13,ohne daß durch die in den Schaftkopf 1 eingeleiteten Kräfte Dreh- oder Schwenkbewegungen innerhalb des Oberschenkelknochens 13 ausgeführt werden können. Die Rillen 57 verlaufen nicht nur parallel zueinander, sondern sind auch geradlinig ausgerichtet.

Wenn der Schaftteil in einen Oberschenkelknochen 13 eingesetzt werden soll, wird zunächst eine entsprechend große Ausnehmung im Bereich der Markhöhle vorbereitet. Sodann wird der Schaft 2 in die vorbereitete Markhöhle eingesetzt. Dabei ist sowohl die Zugstange 29 als auch der Schild 3o mit ihren jeweiligen unteren Enden 32, 46 soweit wie möglich in die Höhlung 21 eingefahren. Der Schild 3o liegt mit seinen Auflageflächen 5o, 51 auf den Druckflächen 52, 53 in einem Bereich auf, der sich in Richtung auf das Ende 46 des Schildes 3o unterhalb der schiefen Ebenen 54, 55 erstreckt.

Nachdem die Rillen 57 mit ihren scharfen Kanten 59 und die Zähne 12 mit ihren unteren Flächen 14 sich fest im Oberschenkelknochen 13 verankert haben, wird die Spannmutter 41 in Richtung auf die elastische Zwischenlage 39 verschraubt, so daß ein Zug auf die Zugstange 29 ausgeübt wird. Diese gleitet durch die Höhlung 21 in Richtung auf den Kragen 3 und nimmt dabei den Schild 3o über die Mitnehmerraste 31 mit. Der Schild 3o läuft mit seinen Auflageflächen 5o, 51 über die Druckflächen 52, 53 und gelangt in den Bereich der schiefen Ebenen 54, 55. Die schiefen Ebenen 54, 55 heben den Schild 3o in Richtung auf die den Zähnen 43 benachbarte Markhöhle ab, so daß sich die Zähne beim weiteren Anziehen der Spannmutter 41 fest in der Markhöhle des Oberschenkelknochens 13 verkrallen. Auf diese Weise wird eine Lockerung des Schildes 3o vermieden und der Schaft 2 in der Markhöhle verspannt. Durch diese Verspannung sind die über den äußeren Krümmungsradius 11 in den Oberschenkelknochen 13 eingeleiteten Kräfte etwa gleich groß denjenigen, die über die inneren Krümmungsradius in den Oberschenkelknochen 13 eingeleitet werden. Sollte die Zugstange 29 mit ihrem oberen Ende 34 zu weit aus dem Kragen 3 herausstehen, so wird das überstehende Ende mit einem entsprechenden Werkzeug abgeschnitten. Falls im Rahmen einer Wechseloperation der Schaftteil aus der Markhöhle herausgenommen werden muß, kann die Spannmutter 41 von der Zugstange 29 abgeschraubt und diese in die Höhlung 21 zurückgestoßen werden. Sodann kann der Schaftteil aus der Markhöhle herausgezogen werden, so daß die Zugstange 29 mit ihrem oberen Ende 34 durch die Bohrung 35 hindurchgleitet.

Sollten während des Verspannens der Zugstange 29 Bewegungen der Zugstange um ihr oberes Ende 34 notwendig werden, so gestattet die in Form eines Kegels ausgebildete Bohrung 35 derartige Pendelbewegungen der Zugstange 29. Darüber hinaus können Verformungen im Bereich der Spannmutter 41 durch die elastische Zwischenlage 39 aufgenommen werden.

Die Ausnehmung 19 kann auch als eine in Richtung auf die Seite 7 des Schaftes 2 geschlossene Bohrung ausgebildet sein. Dabei ist lediglich wichtig, daß die Höhlung an ihrem unteren Ende 22 aufgrund der Ausbildung des äußeren Krümmungsradius 11 soweit offen ist, daß eine Verschiebung der Mitnehmerraste 31 möglich ist. Diese Verschiebung muß über eine Länge stattfinden können, die eine Verspannung des Schildes 3o innerhalb der Markhöhle gestattet.

**Patentansprüche**

1. Schaftteil eines Hüftgelenkes mit einem im wesentlichen kugeligem Schaftkopf (1) und einem sich über einen Kragen (3) an diesen anschließenden Schaft (2), der im Anschluß an den Kragen (3) in Richtung auf sein unteres Ende (9) eine Krümmung (8) aufweist und mittels einer als Zugstange (29) ausgebildeten Spannvorrichtung (28) in einer Markhöhle eines Oberschenkelknochens (13) befestigbar ist, dadurch gekennzeichnet, daß der Schaft (2) in seiner Oberfläche sich in seiner Längsrichtung geradlinig erstreckenden Rillen (57) zur formschlüssigen Verbindung mit der die Schaftoberfläche umgebenden Knochenmasse aufweist, und daß der Schaft (2) an seinem dem Kragen (3) benachbarten oberen Ende in der Markhöhle mit der als Zugstange (29) ausgebildeten Spannvorrichtung (28) verspannbar ist, welche Spannvorrichtung im gespannten Zustand gegenüber dem Schaft (2) einerseits und der Markhöhle andererseits abstützbar ist.

2. Schaftteil nach Anspruch 1, dadurch gekennzeichnet, daß die Zugstange (29) zur Aufbringung einer Spannkraft ein Spannende aufweist, das sich durch eine Bohrung (35) des Kragens (3) erstreckt, auf dessen dem Schaft (2) abgewandten Seite sich eine auf das Spannende aufgeschraubte Spannmutter (41) abstützt.

3. Schaftteil nach Anspruch 2, dadurch gekennzeichnet, daß die Bohrung (35) einen sich in Richtung auf das Ende des Schaftes (2) in Form eines Kegels erweiternden Längsschnitts aufweist.

4. Schaftteil nach Anspruch 2 und 3, dadurch gekennzeichnet, daß die Spannmutter (41) auf einer sie großflächig abstützenden Sitzfläche (37) aufliegt, die quer zur Längsrichtung der Zugstange (29) in den Kragen (3) eingelassen ist.

5. Schaftteil nach Anspruch 4, dadurch gekennzeichnet, daß zwischen der Sitzfläche (37) und der Spannmutter (41) eine elastische Zwischenlage (39) angeordnet ist.

6. Schaftteil nach Anspruch 5, dadurch gekennzeichnet, daß die Sitzfläche (37) am Ende einer im Kragen (3) angeordneten Vertiefung vorgesehen ist, die sowohl die Zwischenlage (39) als auch die Spannmutter (41) aufnimmt.

7. Schaftteil nach Anspruch 1 bis 6, dadurch gekennzeichnet, daß die Zugstange (29) sich durch eine Ausnehmung (19) des Schaftes (2) erstreckt, die in desssen Längsrichtung läuft.

8. Schaftteil nach Anspruch 7, dadurch gekennzeichnet, daß die Ausnehmung (19) als eine Höhlung (21) ausgebildet ist, die einen im wesentlichen U-förmigen Querschnitt aufweist, und an einer ihr zugewandten Außenfläche (58) des Schaftes (2) offen ist.

9. Schaftteil nach Anspruch 8, dadurch gekennzeichnet, daß die Höhlung (21) die Zugstange (29) aufnimmt.

10. Schaftteil nach Anspruch 8 und 9, dadurch gekennzeichnet, daß in der Höhlung (21) eine die Zugstange (29) bei ihrer Verspannung gegen die Markhöle drückende schiefe Ebene ausgebildet ist, die sich auf einem der Außenfläche (58) des Schaftes (2) abgewandten Boden der Höhlung erhebt und eine Steigung in Richtung des Schaftkopfes (1) aufweist.

11. Schaftteil nach Anspruch 8 bis 10, dadurch gekennzeichnet, daß auf der Zugstange (29) eine der auf dem Boden der Höhlung (21) ausgebildeten schiefen Ebene zugewandte schiefe Ebene vorgesehen ist, und die Steigungen der schiefen Ebenen sich einander entsprechen.

12. Schaftteil nach Anspruch 7 bis 11, dadurch gekennzeichnet, daß die Ausnehmung (19) als eine Bohrung ausgebildet ist, die sich unmittelbar an die im Kragen (3) vorgesehe Bohrung (35) anschließt und in Richtung des Endes (9) des Schaftes (2) unmittelbar unterhalb der Krümmung (8) als eine an der Oberfläche des Schaftes (2) offene Höhlung ausgebildet ist.

13. Schaftteil nach Anspruch 1 bis 12, dadurch gekennzeichnet, daß die Zugstange (29) auf ihrer dem Schaft (2) abgewandten Seite eine der Markhöhle zugewandte Druckfläche aufweist.

14. Schaftteil nach Anspruch 13, dadurch gekennzeichnet, daß die Druckfläche in Form von Erhebungen ausgebildet ist, von denen jede eine der Markhöhle zugewandte scharfe Schneidkante aufweist.

15. Schaftteil nach Anspruch 14, dadurch gekennzeichnet, daß die Erhebungen als Zähne ausgebildet sind, deren Schneidkanten aus einer der Markhöhle zugewandten Oberfläche der Zugstange (29) herausragen.

16. Schaftteil nach Anspruch 15, dadurch gekennzeichnet, daß die Zähne sich quer zur Längsrichtung der Zugstange (29) über deren Breite erstrecken.

17. Schaftteil nach Anspruch 15, dadurch gekennzeichnet, daß die Zähne unmittelbar aufeinanderfolgend sich über die gesamte als Druckfläche ausgebildete Oberfläche der Zugstange (29) erstrecken.

18. Schaftteil nach Anspruch 15, dadurch gekennzeichnet, daß die Zähne (43) auf einem Schild (30) ausgebildet sind, daß sich auf der Druckfläche der Zugstange (29) erstreckt.

19. Schaftteil nach Anspruch 18, dadurch gekennzeichnet, daß der Schild (30) auf einer Mitnehmerraste (31) ruht, die an einem der Spannmutter (41) abgewandten unteren Ende (32) der Zugstange (29) befestigt ist und aus der Höhlung (21) herausragt.

20. Schaftteil nach Anspruch 18 und 19, dadurch gekennzeichnet, daß der Schild (30) sich mit seiner der Zugstange (29) zugewandten Rückseite auf Auflageglächen (50,51) abstützt, die beidseits der Zugstange (29) auf einer den Schaft (2) begrenzenden Außenfläche ausgebildet sind.

21. Schaftteil nach Anspruch 20, dadurch gekennzeichnet, daß jede der beiden Auflageflächen mindestens eine sich in Richtung auf den Kragen (3) erhebende schiefe Ebene (54,55) aufweist, auf der sich der Schild (30) mit Druckflächen (52,53) abstützt.

22. Schaftteil nach Anspruch 21, dadurch gekennzeichnet, daß die Druckflächen (52,53) auf einer dem Schaft (2) zugewandten Rückseite des Schildes (30) ausgebildet sind und in Form von schiefen Ebenen ausgebildet sind, die sich den schiefen Ebenen (54,55) anpassen, die auf den Auflageflächen (50,51) ausgebildet sind.

23. Schaftteil nach Anspruch 22, dadurch gekennzeichnet, daß die auf den Druckflächen (52,53) des Schildes (30) ausgebildeten schiefen Ebenen eine Steigung in Richtung auf ein unteres Ende (46) des Schildes (30) aufweisen, das der Spannmutter (41) abgewandt ist.

24. Schaftteil nach Anspruch 23, dadurch gekennzeichnet, daß der Schild (30) an seinem seinem unteren Ende (46) abgewandten oberen Ende (47) eine Öse (48) aufweist.

25. Schaftteil nach Anspruch 22 bis 24, dadurch gekennzeichnet, daß der Schild (30) eine Wölbung in Richtung auf den Schaft (2) aufweist und in Richtung auf sein unteres Ende (46) in Form eines Schildes enger werdend zuläuft.

26. Schaftteil nach Anspruch 1 bis 25, dadurch gekennzeichnet, daß der Kragen (3) auf seiner dem Schaft (2) zugewandten Unterseite mit Schneidkanten versehen ist, die einer Auflagefläche (56) zugewandt sind, auf der der Kragen (3) mit seiner Unterseite aufliegt.

27. Schaftteil nach Anspruch 26, dadurch gekennzeichnet, daß die Schneidkanten als Zähne (12) ausgebildet sind.

28. Schaftteil nach Anspruch 27, dadurch gekennzeichnet, daß jeder Zahn (12) eine Längserstreckung aufweist, die quer zur Richtung einer von der Krümmung (8) des Schaftes (2) aufgespannten gedachten Ebene verläuft.

29. Schaftteil nach Anspruch 27 und 28, dadurch gekennzeichnet, daß jeder Zahn (12) auf seiner einem inneren Krümmungsradius 10 der Krümmung (8) zugewandten Flanke einen steileren Anstieg aufweist als auf seiner anderen Flanke.

**Claims**

1. A femoral component of a hip joint comprising a substantially spherical femoral head (1) and a shank (2) which is connected thereto by way of a collar (3) and which adjoining the collar (3) has a curvature (8) in a direction towards its lower end (9) and which can be fixed in a marrow cavity of a femur (13) by means of a clamping device (28) in the form of a tie rod (29), characterised in that in its surface the shank (2) has grooves (27) extending rectilinearly in its longitudinal direction for positive connection to the bone material surrounding the shank surface, and that at its upper end adjacent the collar (3) the shank (2) can be braced in the marrow cavity with the clamping device (28) which is in the form of a tie rod (29), which clamping device in the tightened condition can be supported relative to the shank (2) on the one hand and the marrow cavity on the other hand.

2. A femoral component according to claim 1 characterised in that the tie rod (29), for applying a clamping force, has a clamping end which extends through a bore (35) in the collar (3), a clamping nut (41) which is screwed on to the clamping end bearing against the side of the collar which is remote from the shank (2).

3. A femoral component according to claim 2 characterised in that the bore (35) is of a longitudinal section which enlarges in the form of a cone in a direction towards the end of the shank (2).

4. A femoral component according to claims 2 and 3 characterised in that the clamping nut (41) lies on a seat surface (37) which supports it over a large area and which is let into the collar (3) transversely to the longitudinal direction of the tie rod (29).

5. A femoral component according to claim 4 characterised in that an elastic intermediate member (39) is disposed between the seat surface (37) and the clamping nut (41).

6. A femoral component according to clam 5 characterised in that the seat surface (37) is provided at the end of a recess which is disposed in the collar (3) and which accommodates both the intermediate member (39) and also the clamping nut (41).

7. A femoral component according to claims 1 to 6 characterised in that the tie rod (29) extends through an opening (19) in the shank (2), which extends in the longitudinal direction of the latter.

8. A femoral component according to claim 7 characterised in that the opening (19) is in the form of a cavity (21) which is of substantially U-shaped cross-section and is open at an outside surface (58), which is towards it, of the shank (2).

9. A femoral component according to claim 8 characterised in that the cavity (21) accommodates the tie rod (29).

10. A femoral component according to claims 8 and 9 characterised in that provided in the cavity (21) is an inclined plane which presses the tie rod (29) against the marrow cavity when the tie rod is tightened and which rises on a floor of the cavity (21) remote from the outside surface (58) of the shank (2) and which has an inclination in the direction of the femoral head (1).

11. A femoral component according to claims 8 to 10 characterised in that provided on the tie rod (29) is an inclined plane which faces towards the inclined plane provided on the floor of the cavity (21), and the inclinations of the inclined planes correspond to each other.

12. A femoral component according to clams 7 to 11 characterised in that the opening (19) is in the form of a bore which immediately adjoins the bore (35) provided in the collar (3) and is in the form of a cavity which is open at the surface of the shank (2) immediately beneath the curvature (8) in the direction of the end (9) of the shank (2).

13. A femoral component according to claims 1 to 12 characterised in that the tie rod (29) has a pressure surface which is towards the marrow cavity, on the side of the tie rod which is remote from the shank (2).

14. A femoral component according to claim 13 characterised in that the pressure surface is in the form of raised portions, each of which has a sharp cutting edge which is towards the marrow cavity.

15. A femoral component according to claim 14 characterised in that the raised portions are in the form of teeth, the cutting edges of which project out of a surface of the tie rod (29), which is towards the marrow cavity.

16. A femoral component according to claim 15 characterised in that the teeth extend transversely to the longitudinal direction of the tie rod (29) over the width thereof.

17. A femoral component according to claim 15 characterised in that the teeth extend in immediate succession over the entire surface of the tie rod (29), which is in the form of a pressure surface.

18. A femoral component according to clam 15 characterised in that the teeth (43) are arranged on a plate member (30) which extends on the pressure surface of the tie rod (29).

19. A femoral component according to claim 18 characterised in that the plate member (30) rests on an entrainment detent (31) which is fixed to a lower end (32), which is remote from the clamping nut (41), of the tie rod (29), and projects out of the cavity (21).

20. A femoral component according to claims 18 and 19 characterised in that the plate member (30) is supported with its rear side towards the tie rod (29) on contact surfaces (50, 51) which are provided on both sides of the tie rod (29) on an outside surface which defines the shank (2).

21. A femoral component according to claim 20 characterised in that each of the two contact surfaces has at least one inclined plane (54, 55) which rises in a direction towards the collar (3) and against which the plate member (30) is supported with pressure surfaces (52, 53).

22. A femoral component according to claim 21 characterised in that the pressure surfaces (52, 53) are provided on a rear side of the plate member (30), which is towards the shank (2), and are in the form of inclined planes which are adapted to the inclined planes (54, 55) provided on the contact surfaces (50, 51).

23. A femoral component according to claim 22 characterised in that the inclined planes which are provided on the pressure surfaces (52, 53) of the plate member (30) have an inclination in a direction towards the lower end (46) of the plate member (30), which is remote from the clamping nut (41).

24. A femoral component according to claim 23 characterised in that the plate member (30) has an eye (48) at its upper end (47) which is remote from its lower end (46).

25. A femoral component according to claims 22 to 24 characterised in that the plate member (30) has a curvature in a direction towards the shank (2) and converges in a narrowing configuration in a direction towards its lower end (46) in the form of a plate member.

26. A femoral component according to claims 1 to 25 characterised in that, on its underside which is towards the shank (2) the collar (3) is provided with cutting edges which are towards a contact surface (56) against which the collar (3) bears with its underside.

27. A femoral component according to claim 26 characterised in that the cutting edges are in the form of teeth (12).

28. A femoral component according to claim 27 characterised in that each tooth (12) is of a longitudinal extent which extends transversely to the direction of a notional plane defined by the curvature (8) of the shank (2).

29. A femoral component according to claims 27 and 28 characterised in that each tooth (12) has a steeper rise on its flank which is towards an inner radius of the curvature (8), than on its other flank.

## Revendications

1. partie tige d'une articulation de hanche, comprenant une tête sensiblement sphérique (1) et une tige (2) qui se raccorde à cette tête par l'intermédiaire d'une collerette (3) et qui, à la suite de la collerette (3) présente une courbure (8) en direction de son extrémité inférieure (9), et peut être fixée, au moyen d'un dispositif de serrage (28) réalisé sous la forme d'un tirant (29), dans le canal médullaire d'un fémur (13), caractérisée en ce que la tige (2) présente, dans sa surface, des rainures (57) s'étendant en ligne droite dans sa direction longitudinale, destinées à établir une liaison par sûreté de forme avec la masse osseuse qui entoure la surface de la tige, et en ce que la tige (2) peut être bloquée dans le canal médullaire à son extrémité supérieure voisine de la collerette (3), au moyen du dispositif de serrage (28) réalisé sous la forme d'un tirant (29), lequel dispositif de serrage peut prendre appui, dans l'état serré contre la tige (2) d'une part et contre le canal médullaire, d'autre part.

2. Partie tige selon la revendication 1, caractérisée en ce que le tirant (29) servant à appliquer une force de serrage présente une extrémité de serrage qui s'étend à travers un perçage (35) de la collerette (3), un écrou de serrage (41) vissé sur l'extrémité de serrage prenant appui sur la surface du perçage qui est à l'opposé de la tige (2).

3. Partie tige selon la revendication 2, caractérisée en ce que le perçage (35) présente une fente longitudinale qui s'élargit en forme de cône en direction de l'extrémité de la tige (2).

4. Partie tige selon l'une des revendications 2 et 3, caractérisée en ce que l'écrou de serrage (41) prend appui sur une surface de portée (37) qui le soutient par une grande surface et qui est formé dans la collerette (3) transversalement à la direction longitudinale du tirant (29).

5. Partie tige selon la revendication 4, caractérisée en ce qu'une couche intercalaire élastique (39) est interposée entre la surface de portée (37) et l'écrou de serrage (41).

6. Partie tige selon la revendication 5, caractérisée en ce que la surface de portée (37) est prévue à l'extrémité d'une chambre ménagée dans la collerette (13), et qui reçoit à la fois la couche intercalaire (39) et l'écrou de serrage (41).

7. Partie tige selon l'une des revendications 1 à 6, caractérisé en ce que le tirant (29) s'étend à travers un évidement (19) de la tige (2) qui s'étend dans sa direction longitudinale.

8. Partie tige selon la revendication 7, caractérisée en ce que l'évidement (19) est constitué par une gorge (21) qui présente une section transversale sensiblement en U et est ouverte sur une surface extérieure (58) de la tige (2) qui est dirigée vers elle.

9. Partie tige selon la revendication 8, caractérisée en ce que la gorge (21) reçoit le tirant (29).

10. Partie tige selon l'une des revendications 8 et 9, caractérisée en ce que, dans la gorge (21), est formé un plan incliné qui presse le tirant (29) contre le canal médullaire lorsqu'on le serre, plan qui s'élève sur un fond de la gorge qui est le plus éloigné de la surface externe (58) de la tige (2) et présente une pente montante en direction de la tête (1) de la tige.

11. Partie tige selon l'une des revendications 8 à 10, caractérisée en ce que, sur le tirant (29), est prévu un plan incliné dirigé vers le plan incliné formé sur le fond de la gorge (21) et les pentes montantes des plans inclinés se correspondent mutuellement.

12. Partie tige selon l'une des revendications 7 à 11, caractérisée en ce que l'évidement (19) est constitué par un perçage qui se raccorde directement au perçage (35) prévu dans la collerette (3) et est formé, en direction de l'extrémité (9) de la tige (2), immédiatement au-dessous de la courbure (8), sous la forme d'une gorge s'ouvrant dans la surface de la tige (2).

13. Partie tige selon l'une des revendications 1 à 12, caractérisée en ce que le tirant (29) présente une surface de pression dirigée vers le canal médullaire dans sa partie qui est à l'opposé de la tige (2).

14. Partie tige selon la revendication 13, caractérisée en ce que la surface de pression présente la forme de protubérances dont chacune présente une arête tranchante vive, dirigée vers le canal médullaire.

15. Partie tige selon la revendication 14, caractérisée en ce que les protubérances sont constituées par des dents dont les arêtes tranchantes font saillie sur la surface du tirant (29) qui est dirigée vers le canal médullaire.

16. Partie tige selon la revendication 15, caractérisée en ce que les dents s'étendent transversalement à la direction longitudinale du tirant (29) sur la largeur de ce dernier.

17. Partie tige selon la revendication 15, caractérisée en ce que les dents s'étendent, directement à la suite l'une de l'autre, sur toute la surface du tirant (29) qui forme une surface de pression.

18. Partie tige selon la revendication 15, caractérisée en ce que les dents (43) sont formées sur une plaque (30) qui s'étend sur la surface de pression du tirant (29).

19. Partie tige selon la revendication 18, caractérisée en ce que la plaque (30) repose sur un mentonnet d'entraînement (31) qui est fixé à une extrémité inférieure (32) du tirant (29) qui est la plus éloignée de l'écrou de serrage (41), et émerge de la gorge (21).

20. Partie tige selon l'une des revendications 18 et 19, caractérisée en ce que la plaque (30) prend appui par sa face arrière dirigée vers le tirant (29) sur des surfaces de portée (50, 51) qui sont formées des deux côtés du tirant (29) sur une surface externe qui délimite la tige (2).

21. Partie tige selon la revendication 20, caractérisée en ce que chacune des deux surfaces de portée présente au moins un plan incliné (54, 55) qui s'élève vers la collerette (3), sur lequel la plaque (30) prend appui par des surfaces de pression (52, 53).

22. Partie tige selon la revendication 21, caractérisée en ce que les surfaces de pression (52, 53) sont formées sur le dos de la plaque (30) qui est dirigée vers la tige (2) et sont présentées sous la forme de plans inclinés qui sont adaptés aux plans inclinés (54, 55) qui sont formés sur les surfaces de portée (50, 51).

23. Partie tige selon la revendication 22, caractérisée en ce que les plans inclinés formés sur les surfaces de pression (52, 53) de la plaque (30) présentent une pente montante en direction de l'extrémité inférieure (46) de la plaque (30) qui est la plus éloignée de l'écrou de serrage (41).

24. Partie tige selon la revendication 23, caractérisée en ce que la plaque (30) présente un oeillet (48) à son extrémité supérieure (47) qui est la plus éloignée de son extrémité inférieure (46).

25. Partie tige selon l'une des revendications 22 à 24, caractérisée en ce que la plaque (30) présente un bombé en direction de la tige (2) et se rétrécit en direction de son extrémité inférieure (46) pour présenter la forme d'un écusson.

26. Partie tige selon l'une des revendications 1 à 25, caractérisée en ce que la collerette (3) est munie, sur sa face inférieure dirigée vers la tige (2), d'arêtes tranchantes qui sont dirigées vers une surface de portée (56) et est appuyée sur celle de la collerette (3) par sa face intérieure.

27. Partie de tige selon la revendication 26, caractérisée en ce que les arêtes tranchantes forment des dents (12).

28. Partie tige selon la revendication 27, caractérisée en ce que chaque dent (12) présente une dimension longitudinale qui s'étend transversalement à la direction d'un plan imaginaire sous-tendu par la courbure (8) de la tige (2).

29. Partie tige selon l'une des revendications 27 et 28, caractérisée en ce que chaque dent (12) présente sur son flanc dirigé vers un rayon de courbure intérieur (10) de la courbure (8), une pente montante plus raide que sur son autre flanc.

Fig.1

Fig. 2

Fig. 3

Fig. 4

Fig. 5

Fig. 6

Fig. 7